# EUROPÄISCHE PATENTSCHRIFT

(11) **EP 2 403 362 B1**
(45) Veröffentlichungstag und Bekanntmachung des Hinweises auf die Patenterteilung: **23.01.2013**
(21) Anmeldenummer: 10707016.1
(22) Anmeldetag: 04.03.2010
(51) Int. Cl.: A23L 1/275, A23K 1/16, C07C 403/24

(54) **PULVERFÖRMIGE ZUSAMMENSETZUNGEN VON ASTAXANTHIN-DERIVATEN II**
POWDERED COMPOSITIONS OF ASTAXANTHIN DERIVATIVES II
COMPOSITIONS PULVÉRULENTES DE DÉRIVÉS D'ASTAXANTHINE II

(30) Priorität: 05.03.2009 EP 09154442
(43) Veröffentlichungstag der Anmeldung: 11.01.2012
(73) Patentinhaber: BASF SE, 67056 Ludwigshafen (DE)
(72) Erfinder: FELDTHUSEN JENSEN, Jesper, 55268 Nieder-Olm (DE); KÖPSEL, Christian, 69469 Weinheim (DE); END, Lutz, 68526 Ladenburg (DE); ENGEL, Robert, 67346 Speyer (DE); BRANDS, Mario, 67063 Ludwigshafen (DE); PELLETIER, Wolf, 76879 Ottersheim (DE); GOTTSCHALK, Thomas, 68165 Mannheim (DE)
(86) Internationale Anmeldenummer: PCT/EP2010/052759
(87) Internationale Veröffentlichungsnummer: WO 2010/100229

(56) Entgegenhaltungen:
- EP-A2- 1 213 013
- EP-A2- 1 228 705
- WO-A1-00/66665
- WO-A1-03/066583
- WO-A2-2005/075385

## Beschreibung

Die vorliegende Erfindung betrifft pulverförmige Zusammensetzungen von Astaxanthin-Derivaten sowie die Verwendung der Zusammensetzungen zur Herstellung von Lösungen des Astaxanthinderivats in für Nahrungsmittel geeigneten Ölen sowie zur Herstellung von Futtermitteln. Die Erfindung betrifft auch ein Verfahren zur Herstellung derartiger Zusammensetzungen und ein Verfahren zur Herstellung von Futtermitteln unter Verwendung der pulverförmigen Zusammensetzungen.

Astaxanthin (3,3'-Dihydroxy-β,β-carotin-4,4'-dion) und seine Derivate, insbesondere seine Ester, sind als Nahrungs- und Futtermittelzusatzstoffe von Interesse. So wird Astaxanthin in großem Umfang bei der Aufzucht von Speisefischen in Aquakulturen (Fischfarmen) als Futtermittelzusatz eingesetzt. Aufgrund der vitaminartigen Eigenschaft von Astaxanthin und seinen Derivaten wirken sie sich vorteilhaft auf die Gesundheit der Fische in den Aquakulturen aus und fördern deren Fruchtbarkeit. Zudem bewirken Astaxanthin und seine Derivate eine Intensivierung der Färbung von Fleisch und Haut der Fische, was nicht zuletzt aus ästhetischen und kulinarischen Gründen wünschenswert ist.

Problematisch sind die geringe Wasserlöslichkeit von Astaxanthin und seinen Derivaten und ihre damit einhergehende schlechte Bioverfügbarkeit. Aus diesem Grund können Astaxanthin und seine Derivate nicht als solche eingesetzt werden, sondern müssen in eine Formulierung überführt werden, die eine hinreichende Bioverfügbarkeit dieser Substanzen gewährleistet. Aufgrund der chemischen Instabilität vom Astaxanthin und seinen Derivaten stellt jedoch die Formulierung dieser Verbindungen eine besondere Herausforderung dar.

Für verschiedene Anwendungszwecke, insbesondere zur Futtermittelherstellung, hat es sich grundsätzlich als vorteilhaft erwiesen, Astaxanthin oder seine Derivate in Form von verdünnten Lösungen in für Nahrungsmittel geeigneten, flüssigen Ölen bereitzustellen. Diese verdünnten Lösungen können dann bei der Futtermittelherstellung verwendet werden und gewährleisten eine hinreichende Bioverfügbarkeit in dem Futtermittel. Bei der Herstellung derartiger Lösungen erweist sich die schlechte Lösungskinetik des Astaxanthins als problematisch.

Die EP 65193 beschreibt ein Verfahren zur Herstellung pulverförmiger Carotinoid-Zusammensetzungen mit Partikelgrößen < 0,5 µm, bei dem man das Carotinoid zunächst in einem flüchtigen, mit Wasser mischbaren Lösungsmittel bei erhöhter Temperatur löst, das Carotinoid aus der so erhaltenen Lösung durch rasches Eintragen in eine wässrige Lösung eines quellbaren Schutzkolloids fällt und die so erhaltene Suspension sprühtrocknet. Das pulverförmige Carotinoid ist in Wasser dispergierbar. Ölige Lösungen werden nicht beschrieben.

Die WO 00/66665 beschreibt ein ähnliches Verfahren zur Herstellung von in Wasser dispergierbaren Trockenpulvern des Astaxanthins. Als Schutzkolloid enthalten die Trockenpulver Casein. Die EP 1228705 und EP 1219292 beschreiben in Wasser dispergierbare Trockenpulver des Astaxanthins ähnlich denen der WO 00/66665, die als Schutzkolloid ein Soja-Protein enthalten. Eigene Untersuchungen der Erfinder der vorliegenden Anmeldung haben gezeigt, dass bei direkter Einarbeitung der dort beschriebenen Trockenpulver in für Nahrungsmittel geeignete Öle das Astaxanthin nicht oder nur zu geringen Anteilen in die Ölphase übergeht.

Die EP 845503 beschreibt flüssige mit Öl mischbare Carotinoid-Zubereitungen in Form einer Wasser-in-Öl-Emulsion, worin die emulgierte wässrige Phase schutzkolloidstabilisierte Carotinoid-Partikel in dispergierter Form enthält. Das Verfahren zur Herstellung dieser Zubereitungen ist vergleichsweise aufwändig. Zudem ist der Anteil an Carotinoid in der Emulsion vergleichsweise gering. Außerdem geht das Astaxanthin beim Verdünnen der Zubereitung mit einem für Nahrungsmittel geeigneten Öl nur teilweise in die Ölphase über.

Die WO 03/102116 beschreibt ölige Lösungen von Carotinoiden wie Astaxanthin. Ihre Herstellung erfolgt durch Lösen des Carotinoids in einem organischen Lösungsmittel wie N-Methylpyrrolidon in Anwesenheit eines lipophilen Dispergiermittels und Entfernen des Lösungsmittels. Das erhaltene Pulver wird dann in einem Öl, beispielsweise Fischöl, gelöst. Dieses Verfahren ist vergleichsweise aufwändig und bedarf größerer Mengen an Schutzkolloiden.

Die WO 2006/125591 beschreibt die Herstellung von öligen Carotinoid-Lösungen, beispielsweise Lösungen des Astaxanthins, wobei man zunächst eine Suspension des Carotinoids in der Ölphase bereitstellt, die Suspension für eine kurze Zeit auf hohe Temperaturen, z. B. im Bereich von 100 bis 230 °C erhitzt, um das Carotinoid in Lösung zu bringen, und die heiße Lösung mit einem kalten Öl vermischt. Zur Herstellung von öligen Lösungen des Astaxanthins werden regelmäßig Temperaturen oberhalb 160 °C angewendet. Dieses Verfahren ist mit einer Reihe von Nachteilen verbunden. Zum einen führt das Erhitzen aufgrund der chemischen Labilität von β-Carotinoiden unter anderem zu einer unerwünschten cis/trans-Isomerisierung der exocyclischen Doppelbindungen und damit zu einem Aktivitätsverlust. Zudem erweist sich die Bereitstellung öliger Suspensionen des Astaxanthins durch Vermahlen von Astaxanthin in Öl als problematisch.

Der vorliegenden Erfindung liegt die Aufgabe zu Grunde, geeignete Formulierungen von Astaxanthin oder einem Astaxanthin-Derivat bereitzustellen, die es erlauben, Astaxanthin oder das Astaxanthin-Derivat direkt in ein für Nahrungsmittel geeignetes Öl einzuarbeiten, ohne dass es der Anwendung von Temperaturen oberhalb 100 °C bedarf oder der Redispergierung in Wasser bedarf. Dabei sollte das Astaxanthin-Derivat weitgehend oder vollständig in die Ölphase übergehen. Die Formulierungen sollten einfach herzustellen sein, und das darin enthaltene Astaxanthin sollte einen hohen Gehalt an all-trans Isomer aufweisen.

Die ältere internationale Patentanmeldung PCT/EP2008/061384 beschreibt ein Verfahren zur Herstellung von öligen Lösungen des Astaxanthins in für Nahrungsmittel geeigneten Ölen, bei denen man bestimmte Astaxanthin-Derivate der im Folgenden angegebenen Formel I zunächst in eine Suspension in einem essbaren Öl überführt, wobei die Partikel des Astaxanthin-Derivats in der Suspension einen volumenmittleren Teilchendurchmesser, bestimmt durch Fraunhofer-Beugung, im Bereich von 0,5 bis 50 µm aufweisen. Diese Suspensionen sind typischerweise bei Temperaturen von 25 °C flüssig und können in einfacher Weise mit flüssigen, für Nahrungsmittel geeigneten Ölen verdünnt werden, wobei sich die verwendeten Astaxanthin-Derivate aufgrund ihrer vorteilhaften Lösungskinetik und der geringen Partikelgröße rasch in dem zur Verdünnung eingesetzten Öl auflösen, ohne dass es der Anwendung von Temperaturen oberhalb 100 °C und/oder größerer Mengen an Emulgatoren bedarf.

Es wurde nun überraschenderweise gefunden, dass Astaxanthin-Derivate, die zu wenigstens 70 Gew.-% aus einer Verbindung der im Folgenden definierten Formel I bestehen, anders als Astaxanthin oder andere Astaxanthin-Derivate, sich besonders einfach in Pulver überführen lassen und zudem eine bessere Löslichkeit in Ölen aufweisen, wenn das Pulver aus den folgenden Bestandteilen besteht:
i) einem Astaxanthin-Derivat, das zu wenigstens 70 Gew.-% aus einer Verbindung der im Folgenden definierten Formel I besteht, in einer Menge von 1 bis 50 Gew.-%, bezogen auf das Gesamtgewicht der Zusammensetzung,
ii) wenigstens einem proteinischen Schutzkolloid in einer Menge von wenigstens 10 bis 70 Gew.-%, bezogen auf das Gesamtgewicht der Zusammensetzung, und
iii) gegebenenfalls einem oder mehreren, für Nahrungsmittel geeigneten Zusatzstoffen in einer Menge von bis zu 89 Gew.-%, bezogen auf das Gesamtgewicht der Zusammensetzung.

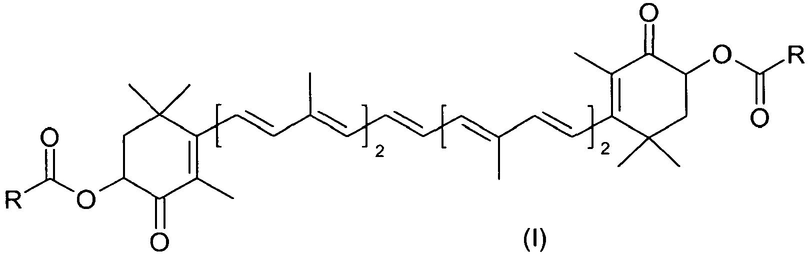

In Formel I steht R für einen Rest der Formel 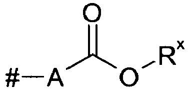 worin # die Anknüpfung an die Carbonylgruppe bedeutet, A für -CH₂CH₂- oder für -CH=CH- steht und Rₓ für C₁-C₄ Alkyl steht.

Dementsprechend betrifft die vorliegende Erfindung eine pulverförmige Zusammensetzung eines Astaxanthin-Derivats, bestehend aus:
i) einem Astaxanthin-Derivat, das zu wenigstens 70 Gew.-%, vorzugsweise wenigstens 80 Gew.-%, insbesondere wenigstens 90 Gew.-% und speziell wenigstens 96 Gew.-% aus einer Verbindung der im Folgenden definierten Formel I besteht, in einer Menge von 1 bis 50 Gew.-%, insbesondere 2 bis 30 Gew.-% und speziell 50 bis 20 Gew.-%, bezogen auf das Gesamtgewicht der Zusammensetzung,
ii) wenigstens einem proteinischen Schutzkolloid in einer Menge von wenigstens 10 bis 70 Gew.-%, insbesondere 15 bis 65 Gew.-% und speziell 20 bis 60 Gew.-%, bezogen auf das Gesamtgewicht der Zusammensetzung, und
iii) gegebenenfalls einem oder mehreren, für Nahrungsmittel geeigneten Zusatzstoffen in einer Menge von bis zu 89 Gew.-%, insbesondere bis 83 Gew.-%, speziell bis 75 Gew.-%, z. B. 10 bis 89 Gew.-%, insbesondere 15 bis 83 Gew.-% und speziell 20 bis 75 Gew.-%, bezogen auf das Gesamtgewicht der Zusammensetzung.

Die Erfindung ist mit einer Reihe von Vorteilen verbunden. Zum einen lassen sich unter Verwendung der erfindungsgemäßen pulverförmigen Zusammensetzungen in einfacher Weise Lösungen von Astaxanthin-Derivaten in für Nahrungsmittel geeigneten Ölen durch Einarbeiten der erfindungsgemäßen Zusammensetzung in das für Nahrungsmittel geeignete Öl herstellen. Anders als in dem in WO 2006/125591 beschriebenen Verfahren, bedarf es hierzu grundsätzlich nicht der Anwendung von Temperaturen oberhalb 100 °C. Vielmehr kann man unter Verwendung der erfindungsgemäßen Formulierungen verdünnte Lösungen des Astaxanthin-Derivats in dem flüssigen Öl bei Temperaturen von unterhalb 100 °C, insbesondere maximal 90 °C oder gar maximal 80 °C, z. B. bei Temperaturen im Bereich von 20 bis 90 °C insbesondere 30 bis 85 °C, herstellen. Aufgrund der niedrigen Temperaturen zeichnen sich die auf diese Weise erhältlichen Lösungen durch einen sehr hohen Anteil an all-trans-Isomeren aus, der in der Regel oberhalb 70 %, häufig oberhalb 80 %, insbesondere oberhalb 90 %, bezogen auf das in der Formulierung enthaltene Astaxanthin-Derivat liegt. Ferner lassen sich unter Verwendung von Astaxanthin-Derivaten, die zu wenigstens 70 Gew.-% aus wenigstens einer Verbindungen der Formel I bestehen, sehr viel einfacher pulverförmige Zusammensetzung von Astaxanthin-Derivaten herstellen als bei Verwendung des Astaxanthins selber oder anderer, davon verschiedener Astaxanthin-Derivate wie Astaxanthindiacetat oder Astaxanthindisuccinat.

Die erfindungsgemäßen Zusammensetzungen haben neben einer guten Verdünnbarkeit in den für Nahrungsmittel geeigneten Ölen den Vorteil einer verbesserten Handhabbarkeit und Stabilität im Vergleich zu den aus PCT/EP2008/061384 bekannten flüssigen Suspensionen.

In Formel I steht C₁-C₄-Alkyl für einen linearen oder verzweigten, gesättigten Kohlenwasserstoffrest mit 1 bis 4 C-Atomen wie Methyl, Ethyl, n-Propyl, 1-Methylethyl, Butyl, 1-Methylpropyl, 2-Methylpropyl oder 1,1-Dimethylethyl.

Die beiden Reste R in Formel I können gleich oder verschieden sein und sind vorzugsweise identisch.

Vorzugsweise steht A für 1,2-Ethandiyl. R^{x} steht vorzugsweise für Methyl oder Ethyl.

Verbindungen der Formel I, worin A im Rest R für 1,2-Ethandiyl steht, werden im Folgenden auch als Dialkyldisuccinate bezeichnet. Hierunter besonders bevorzugt ist das Dimethylsuccinat.

Die Verbindungen der Formel I können, da die die Gruppe O-C(O)R tragenden Kohlenstoffatome Asymmetriezentren sind, diastereomere und enantiomere Formen bilden, nämlich die 3R,3'S-Form, die 3S,3'R-Form, die 3R,3'R-Form und die 3S,3'S-Form. Sofern die Reste R in Formel I gleich sind, sind die 3R,3'S-Form und die 3S,3R-Form identisch und achiral (meso-Form) und die 3S,3'S-Form und die 3R,3'R-Form bilden ein Enantiomerenpaar. Für die Erfindung können sowohl die reinen Enantiomere und Diasteromere sowie Gemische der vorgenannten Diastereomere, z. B. ein racemisches Gemisch der 3S,3'S-Form und der 3R,3'R-Form als auch ein Gemisch der meso-Form mit der 3S,3'S-Form und/oder der 3R,3'R-Form oder ein Gemisch der meso-Form und dem Racemat der 3S,3'S-Form und der 3R,3'R-Form eingesetzt werden. In einer bevorzugten Ausgestaltung der Erfindung wird die Verbindung der Formel I in der 3S,3'S-Form oder einer Mischung der 3S,3'S-Form mit einer oder weiteren Formen der Verbindung der Formel I eingesetzt, worin die 3S,3'S-Form wenigstens 80 Gew.-%, insbesondere wenigstens 90 Gew.-%, bezogen auf die Gesamtmenge der Verbindung der Formel I ausmacht. In einer weiteren bevorzugten Ausgestaltung der Erfindung wird die Verbindung der Formel I in der 3R,3'R-Form oder einer Mischung der 3R,3'R-Form mit einer oder weiteren Formen der Verbindung der Formel I eingesetzt, worin die 3R,3'R-Form wenigstens 80 Gew.-%, insbesondere wenigstens 90 Gew.-%, bezogen auf die Gesamtmenge der Verbindung der Formel I ausmacht.

In den erfindungsgemäßen Zusammensetzungen weist das Astaxanthin-Derivat üblicherweise eine Reinheit, bezogen auf das in Formel I gezeigte all-trans-Isomer, von wenigstens 70 Gew.-%, häufig wenigstens 80 Gew.-%, vorzugsweise wenigstens 90 Gew.-% und insbesondere wenigstens 96 Gew.-% auf. Neben dem all-trans-Isomeren kann das Astaxanthin-Derivat auch Anteile an Astaxanthin-Verbindungen der Formel I enthalten, in denen eine oder mehrere Doppelbindungen der in Formel I dargestellten all-trans-Isomere cis-Konfiguration aufweisen. Die Gesamtmenge an all-trans-Isomer der Formel I und cis-Isomeren der Formel I beträgt in der Regel wenigstens 80 Gew.-% und häufig wenigstens 90 Gew.-%, bezogen auf das in der Zusammensetzung enthaltene Astaxanthin-Derivat. Neben dem all-trans-Isomeren der Formel I und etwaigem cis-Isomeren kann das Astaxanthin-Derivat auch weitere Carotinoide umfassen, wobei der Anteil dieser Verunreinigungen in der Regel 30 Gew.-%, häufig 20 Gew.-%, insbesondere 10 Gew.-%, besonders bevorzugt 4 Gew.-%, bezogen auf die Gesamtmenge des Astaxanthin-Derivats nicht überschreitet. Bei diesen Verunreinigungen handelt es sich in erster Linie um Halbester des Astaxanthins, d. h. um Verbindungen der im Folgenden gezeigten Formel II 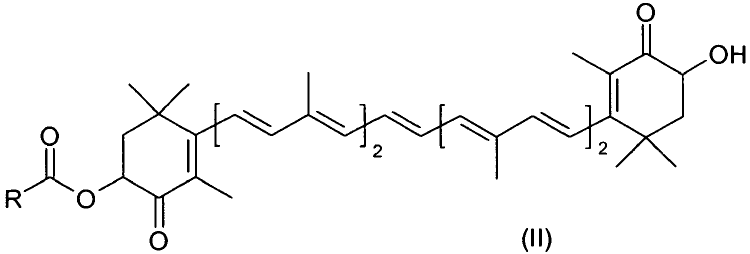 worin R die zuvor genannten Bedeutungen hat bzw. um cis-Isomere davon, sowie um Astaxanthin selber, Adonirubin und/oder Semi-Astacin.

Insbesondere enthält die erfindungsgemäße Zusammensetzung ein Astaxanthin-Derivat, das die an ein für Nahrungsmittel (d. h. für Lebens- und/oder Futtermittel) zugelassenes Astaxanthin gestellten Anforderungen erfüllt, d. h. das weniger als 4 Gew.-% Carotinoide, die von Astaxanthin und seinen Derivaten verschieden sind, enthält und das einen Schwermetallgehalt von höchstens 10 ppm aufweist, das zu wenigstens 70 Gew.-%, häufig zu wenigstens 80 Gew.-%, vorzugsweise zu wenigstens 90 Gew.-%, insbesondere zu wenigstens 96 Gew.-%, bezogen auf die Gesamtmenge an Astaxanthin-Derivat in der Zusammensetzung, aus einer Astaxanthin-Verbindung der Formel I besteht,, wobei das in der Zusammensetzung enthaltene Astaxanthin-Derivat zu wenigstens 70 Gew.-%, häufig wenigstens 80 Gew.-%, vorzugsweise wenigstens 90 Gew.-% und insbesondere wenigstens 95 Gew.-% aus dem all-trans-Isomeren besteht.

Erfindungsgemäß enthält die Zusammensetzung wenigstens ein proteinisches Schutzkolloid. Hierbei handelt es sich um eine in Wasser lösliche oder quellbare polymere Substanz, die aus Aminosäuren aufgebaut ist und gegebenenfalls glykosidische Bestandteile aufweist. Das proteinische Schutzkolloid ist in der Regel pflanzlichen oder tierischen Ursprungs.

Das Molekulargewicht (Gewichtsmittel) des proteinischen Schutzkolloids liegt typischerweise im Bereich von 10 000 bis 250 000 Dalton, insbesondere im Bereich von 15 000 bis 200 000 Dalton und speziell im Bereich von 20 000 bis 180 000 Dalton.

Das Gewichtsverhältnis von proteinischem Schutzkolloid zu Astaxanthin-Derivat liegt typischerweise im Bereich von 50 : 1 bis 1 : 5, insbesondere im Bereich von 20 : 1 bis 1 : 2 und speziell im Bereich von 10 : 1 bis 1 : 1.

Beispiele für proteinische Schutzkolloide sind Caseine und Caseinate, einschließlich αs1-, αs2, β- und κ-Kasein und deren Gemische, Prolamine, d. h. Speicherproteine aus Getreidesamen wie Gliadin, Secalin, Avenalin, Hordein, Zein, Oryzin und Kafirin, Molkeproteine wie beta-Lactoglobulin, alpha-Lactalbumin, Serumalbumin, Proteosepepton, Immunoglobuline und deren Gemische, Gelatine wie Rinder-, Schweine- und Fischgelatine, insbesondere sauer abgebaute Gelatine wie Gelatine A100 und A200, alkalisch abgebaute Gelatine wie Gelatine B100 und B200 sowie enzymatisch abgebaute Gelatinetypen wie die unter den Handelsbezeichnungen Gelita® Collagel A, Gelita® Sol DA, Gelita® Sol PA und Gelita® Sol LDA (DGF Stoess) erhältlichen Produkte, weiterhin Sojaprotein und teilhydrolysiertes Sojaprotein sowie Lupin-Protein.

Unter einem teilhydrolysierten Sojaprotein versteht man ein Sojaprotein, das einem partiellen enzymatischen Abbau mit einer Protease unterworfen wurde. Der Abbaugrad DH eines solchen teilhydrolysierten Sojaproteins beträgt in der Regel wenigstens 1 %, insbesondere wenigstens 5 %, z. B. im Bereich von 1 bis 20 % und insbesondere im Bereich von vorzugsweise bevorzugt 5 bis 16 %. Unter dem Abbaugrad DH versteht man den Quotienten aus der Anzahl der hydrolysierten Peptidbindungen zur Gesamtzahl der Peptidbindungen im Ausgangsprotein. Der Abbaugrad kann gemäß der so genannten "pH-Stat-Methode" bestimmt werden, wie von C. F. Jacobsen et al. in "Methods of Biochemical Analysis", Vol. IV, S. 171-210, Interscience Publishers Inc., New York 1957, beschrieben.

Als Sojaproteine für die Herstellung der erfindungsgemäßen Zusammensetzungen, aber auch zur Herstellung der teilhydrolysierten Sojaproteine werden üblicherweise handelsübliche Sojaprotein-Isolate und -Konzentrate mit Proteingehalten von in der Regel 70 bis 90 Gew.-% eingesetzt, wobei die restlichen 10 bis 30 Gew.-% mehr oder weniger undefinierte andere Pflanzenbestandteile darstellen.

Unter Lupin-Protein versteht man aus Lupine gewonnene Pflanzenproteine.

Bevorzugte Schutzkolloide sind Casein, Caseinate z. B. Na-Caseinat, Prolamine und Molkenproteine. Insbesondere umfasst das proteinische Schutzkolloid ein Kasein oder ein Gemisch aus Casein und/oder Caseinat und einem weiteren proteinischen Schutzkolloid, z. B. ein Gemisch aus Casein und/oder Caseinat und einem Prolamin, ein Gemisch aus Casein und/oder Caseinat und einem Molkenprotein oder Molkenproteingemisch, ein Gemisch aus Casein und/oder Caseinat und einem Lupin-Protein, ein Gemisch aus Casein und/oder Caseinat und Sojaprotein, ein Gemisch aus Casein und/oder Caseinat und Sojaproteinhydrolysat. Insbesondere bildet das Casein bzw. das Caseinat den Hauptbestandteil des proteinischen Schutzkolloids, der Anteil am Schutzkolloid beträgt wenigstens 50 Gew.-%, insbesondere wenigstens 80 Gew.-% oder wenigstens 90 Gew.-%.

In einer ebenfalls bevorzugten Ausführungsform der Erfindung umfasst das proteinische Schutzkolloid wenigstens ein Molkenprotein oder ein Gemisch aus Molkenprotein und einem weiteren proteinischen Schutzkolloid, z. B. ein Gemisch aus Molkenprotein und Prolamin, ein Gemisch aus Molkenprotein und einem oder mehreren Prolaminen, ein Gemisch aus Molkenprotein und Sojaprotein, ein Gemisch aus Molkenprotein und Sojaproteinhydrolysat. Insbesondere bildet dann das Molkenprotein den Hauptbestandteil des proteinischen Schutzkolloids, der Anteil am Schutzkolloid beträgt wenigstens 50 Gew.-%, insbesondere wenigstens 80 Gew.-% oder wenigstens 90 Gew.-%.

In einer weiteren, ebenfalls bevorzugten Ausführungsform der Erfindung umfasst das proteinische Schutzkolloid wenigstens ein Prolamin oder ein Gemisch aus Prolamin und einem weiteren proteinischen Schutzkolloid, z. B. ein Gemisch aus Prolamin und Lupin-Protein, ein Gemisch aus Prolamin und einem oder mehrerer Molkenproteine, ein Gemisch aus Prolamin und Sojaprotein, ein Gemisch aus Prolamin und Sojaproteinhydrolysat. Insbesondere bildet das dann das Prolamin den Hauptbestandteil des proteinischen Schutzkolloids, der Anteil am Schutzkolloid beträgt wenigstens 50 Gew.-%, insbesondere wenigstens 80 Gew.-% oder wenigstens 90 Gew.-%.

Neben den vorgenannten Bestandteilen kann die erfindungsgemäße Zusammensetzung einen oder mehrere weitere, für Nahrungsmittel geeignete Bestandteile enthalten. Hierzu zählen Oxidationsstabilisatoren (Antioxidantien), Emulgatoren, Konservierungsmittel, Weichmacher, Filmbildner, Füllstoffe, Öle, Überzugsmittel und Puderungsmittel. Der Gesamtmenge dieser Bestandteile beträgt erfindungsgemäß maximal 89 Gew.-%, z. B. 10 bis 89 Gew.-%, bezogen auf das Gesamtgewicht der Zusammensetzung, und wird insbesondere 83 Gew.-% und insbesondere 75 Gew.-%, bezogen auf das Gesamtgewicht der Zusammensetzung nicht überschreiten und liegt dann vorzugsweise im Bereich von 15 bis 83 Gew.-%, insbesondere im Bereich von 20 bis 75 Gew.-%, bezogen auf das Gesamtgewicht der Zusammensetzung.

Beispiele für Antioxidantien sind insbesondere Tocopherole wie α-Tocopherol, α-Tocopherolpalmitat, α-Tocopherolacetat, tert.-Butylhydroxytoluol, tert.-Butylhydroxyanisol, Ascorbinsäure, deren Salze und Ester wie z. B. Natriumascorbat, Calciumascorbat, Ascorbylphosphatester, Fettsäureester der Ascorbinsäure wie Ascorbylstearat und Ascorbylpalmitat und Ethoxyquin. Die Antioxidantien sind, sofern erwünscht, in der erfindungsgemäßen Zusammensetzung typischerweise in Mengen von 0,1 bis 20 Gew.-%, häufig 0,5 bis 10 Gew.-%, bezogen auf das Gesamtgewicht der Zusammensetzung, enthalten.

Beispiele für Emulgatoren sind insbesondere lipophile Dispergiermittel wie beispielsweise solche mit einem HLB-Wert von maximal 10. Beispiele für geeignete Emulgatoren sind Fettsäureester der Ascorbinsäure wie Ascorbylpalmitat, Polyglycerin-Fettsäureester wie Polyglycerin-3-polyrizinoleat (PGPR90), Sorbitanfettsäureester, insbesondere Sorbitan-C₁₀-C₂₈-fettsäureester wie z. B. Mono- und Di-C₁₀-C₂₈-fettsäureester des Sorbitans wie Sorbitanmonolaurat, Sorbitanmonooleat und Sorbitanmonostearat (SPAN60), ethoxilierte Sorbitanfettsäureester wie PEG(20)-Sorbitol-monooleat, Monoester der Milchsäure mit gesättigten C₁₀-C₂₄-Fettsäuren, Zuckerester von gesättigten C₁₆-C₂₈-Fettsäuren, Propylenglycol-Fettsäureester sowie Phospholipide wie Lecithin. Emulgatoren sind, sofern erwünscht, in der erfindungsgemäßen Zusammensetzung typischerweise in Mengen von 0,1 bis 20, häufig 0,5 bis 10 Gew.-%, bezogen auf das Gesamtgewicht der Zusammensetzung, vorzugsweise in einer Menge von 1 bis 30 Gew.-%, insbesondere in einer Menge von 2 bis 15 Gew.-%, bezogen auf das Astaxanthin-Derivat, enthalten.

Beispiele für geeignete Konservierungsmittel sind Benzoesäure und ihre Salze, insbesondere ihre Natrium-, Kalium- und Calciumsalze, 4-Hydroxybenzoesäure (PHB) und ihre Salze, insbesondere ihre Natrium-, Kalium- und Calciumsalze, und ihre Alkylester (PHB-Alkylester), insbesondere ihre Methyl-, Ethyl- und Propylester und die Salze der PHB-Alkylester wie das Natriumsalz des PHB-Methylesters, das Natriumsalz des PHB-Ethylesters und das Natriumsalz des PHB-Propylesters, Sorbinsäure und ihre Salze, insbesondere ihre Natrium-, Kalium- und Calciumsalze, Propionsäure und ihre Salze wie insbesondere ihre Natrium-, Kalium- und Calciumsalze, Borsäure und Milchsäure. Konservierungsmittel sind, sofern erwünscht, in der erfindungsgemäßen Zusammensetzung typischerweise in Mengen von 0,001 bis 10 Gew.-%, häufig 0,01 bis 5 Gew.-%, bezogen auf das Gesamtgewicht der Zusammensetzung, enthalten.

Als Weichmacher kommen insbesondere Mono-, Di- und Oligosaccharide, insbesondere solche mit 3 bis 30 und speziell 5 bis 20 Monosaccharideinheiten, sowie Zuckeralkohole in Betracht, z. B. Saccharose, Glucose, Glucosesirup, Dextrine, insbesondere solche mit 5 bis 20 Glukose-Einheiten (z. B. solche, die unter der Bezeichnung "Glucidex" von der Fa. Rouquette vertrieben werden), Lactose, Invertzucker, Sorbit, Mannit, Glycerin und Mischungen der vorgenannten Substanzen. Bevorzugte Weichmacher sind Mono- und Disaccharide, die vorgenannten Dextrine mit 5 bis 20 Glukose-Einheiten sowie Sorbit und Mannit. In einer bevorzugen Ausführungsform der Erfindung enthält die Zusammensetzung wenigstens einen Weichmacher. Die Weichmacher sind vorzugsweise in einer Menge von 5 bis 70 Gew.-%, insbesondere 10 bis 60 Gew.-% in der Zusammensetzung enthalten. Die Weichmacher wirken in der Regel auch als Filmbildner.

Die erfindungsgemäßen Zusammensetzungen können auch Füllstoffe enthalten. Hierunter versteht man inerte, feste partikelförmige Substanzen, wie beispielsweise feinteilige Kieselsäure, wie pyrogene Kieselsäure oder Kieselgel (E 551), Titandioxid, Hydroxide wie Aluminiumhydroxid, Carbonate und Hydrogencarbonate wie Natriumcarbonat (E 500), Natriumhydrogencarbonat, Kaliumcarbonat (E 501), Magnesiumcarbonat (E 504) und Calciumcarbonat (E 170), Silikate wie Natriumsilikat (E 550), Magnesiumsilikat (E 553a), Talk (E 553b), Calciumsilikat (E 552), Zinksilikat (E 557), Aluminiumsilikate wie Natriumaluminiumsilikat (554), Kaliumaluminiumsilikat (E 555), Calciumaluminiumsilikat (E 556), Bentonit (E 558), Kaolin (E 559), Tricalciumphosphat, Natriumchlorid, inerte, für Nahrungsmittel geeignete feste organische Materialien in Pulverform, z. B. ggf. modifizierte Polysacharide wie Stärke, Cellulose und Methylcellulose. Der Anteil der Füllstoffe wird in der Regel 50 Gew.-%, insbesondere 30 Gew.-% der Zusammensetzung nicht überschreiten. Bevorzugte Füllstoffe sind die vorgenannten Polysacharide.

Die erfindungsgemäßen Zusammensetzungen können auch Öle enthalten. Der Ölanteil liegt, sofern erwünscht, vorzugsweise im Bereich von 0,1 bis 10 Gew.-%, bezogen auf das Gesamtgewicht der Zusammensetzung. Beispiele für Öle sind insbesondere Fettsäureglyceride, die natürlichen, z. B. tierischen oder pflanzlichen, Ursprungs, synthetisch oder semisynthetisch sein können. Beispiele für geeignete Öle sind Pflanzenöle wie Sojabohnenöl, Sonnenblumenöl, Maiskeimöl, Leinsamenöl, Rapsöl, Distelöl, Weizenkeimöl, Reisöl, Kokosöl, Mandelöl, Aprikosenkernöl, Palmöl, Palmkernöl, Avocadoöl, Jojobaöl, Haselnussöl, Walnussöl, Erdnussöl, Pistazienöl und PUFA-Öle (PUFA = mehrfach ungesättigte Fettsäuren (polyunsaturated fatty acids) wie Eikosapentaensäure (EPA), Docosahexaensäure (DHA) und α-Linolensäure), weiterhin semisynthetische Triglyceride, z. B. Caprylsäure/Caprinsäure-Tricyceride wie die Miglyol-Typen, weiterhin tierische Öle und Fette wie Fischöle einschließlich Makrelen-, Sprotten-, Thunfisch-, Heilbutt-, Kabeljau- und Lachsöl und Lanolin. Die Öle sind, sofern erwünscht, in der erfindungsgemäßen Zusammensetzung typischerweise in Mengen von 0,5 bis 30 Gew.-%, häufig 1 bis 20 Gew.-%, bezogen auf das Gesamtgewicht der Zusammensetzung, enthalten.

Beispiele für Überzugsmittel sind Maisstärke, Kieselsäure und Tricalciumphosphat. Ihre Menge beträgt, sofern erwünscht, typischerweise 0,01 bis 10 Gew.-%, bezogen auf das Gesamtgewicht der Zusammensetzung.

In einer bevorzugten Ausführungsform der Erfindung enthält die Zusammensetzung wenigstens einen der vorgenannten Weichmacher, der insbesondere unter den vorgenannten Sacchariden und Zuckeralkoholen, insbesondere unter Lactose, Glucose, Sorbit, Mannit und Dextrinen und deren Gemischen ausgewählt ist.

In einer bevorzugten Ausführungsform der Erfindung enthält die Zusammensetzung wenigstens eines der vorgenannten Antioxidantien, das insbesondere unter den vorgenannten Antioxidantien wie insbesondere Tocopherolen wie α-Tocopherol, α-Tocopherolpalmitat, α-Tocopherolacetat, tert.Butylhydroxytoluol, tert.-Butylhydroxyanisol, Estern der Ascorbinsäure wie z. B. Ascorbylpalmitat und Ascorbylstearat und Ethoxyquin ausgewählt ist.

In einer bevorzugten Ausführungsform der Erfindung enthält die Zusammensetzung wenigstens einen der vorgenannten Emulgatoren, der insbesondere unter den vorgenannten Fettsäureestern der Ascorbinsäure wie Ascorbylpalmitat, Polyglycerin-Fettsäureester wie Polyglycerin-3-polyrizinoleat (PGPR90), Sorbitanfettsäureester, insbesondere Sorbitan-C₁₀-C₂₈-fettsäureester wie z. B. Mono- und Di-C₁₀-C₂₈-fettsäureester des Sorbitans wie Sorbitanmonolaurat, Sorbitanmonooleat und Sorbitanmonostearat (SPAN60), ethoxilierten Sorbitanfettsäureester wie PEG(20)-Sorbitol-monooleat und Propylenglycol-Fettsäureester ausgewählt ist.

Die erfindungsgemäßen Zusammensetzungen sind nicht nur, wie zu erwarten gewesen wäre, in Wasser dispergierbar. Beim unmittelbaren Einarbeiten des Pulvers in für Nahrungsmittel geeignete Öle führen sie überraschenderweise zu einem raschen Auflösen des Astaxanthin-Derivats in dem Öl, was bei Astaxanthin selber nicht möglich ist oder sehr viel langsamer vonstatten geht. Insbesondere werden beim unmittelbaren Einarbeiten des Pulvers in das für Nahrungsmittel geeignete Öl wenigstens 80 %, insbesondere wenigstens 90 % des in der pulverförmigen Zusammensetzung enthaltenen Astaxanthin-Derivats von dem für Nahrungsmittel geeigneten Öl gelöst, ohne dass es längerer Lösezeiten von mehr als einer Stunde und/oder höherer Temperaturen von mehr als 100 °C bedarf.

Die erfindungsgemäßen Pulver bilden beim Dispergieren in Wasser eine feinteilige Dispersion von Partikeln des Astaxanthin-Derivats, die durch das proteinische Schutzkolloid stabilisiert werden. Die Partikelgröße kann, in Abhängigkeit von der Herstellung der Pulver über weite Bereiche variieren, wobei die Partikel in der Regel einen gewichtsmittleren bzw. volumenmittleren Partikeldurchmesser von nicht mehr als 5 µm, häufig nicht mehr als 3 µm und insbesondere nicht mehr als 2 µm aufweisen, z. B. im Bereich von 10 nm bis 5 µm, häufig im Bereich von 20 nm bis 3 µm und insbesondere im Bereich von 50 nm bis 2 µm.

Gemäß einer ersten Ausführungsform, zerfallen die erfindungsgemäßen Pulver beim Dispergieren in Wasser zu feinteiligen Partikeln, wobei die Partikel in der Regel einen gewichtsmittleren bzw. volumenmittleren Partikeldurchmesser von nicht mehr als 5 µm, häufig nicht mehr als 3 µm und insbesondere nicht mehr als 2 µm aufweisen, z. B. im Bereich von 0,1 bis 5 µm, insbesondere im Bereich von 0,2 bis 3 µm oder im Bereich von 0,3 bis 2 µm (bestimmt durch Lichtstreuung, z. B. bei 25 °C in einer 0,01 bis 0,1 gew.-%igen wässrigen Dispersion).

Gemäß einer zweiten Ausführungsform zerfallen die erfindungsgemäßen Pulver beim Dispergieren in Wasser zu Nanopartikeln, d. h. zu Partikeln, deren Partikelgröße (Durchmesser) unterhalb 1 µm liegt. Insbesondere hat es sich als vorteilhaft erwiesen, wenn die wässrige Dispersion der Pulverteilchen in Wasser eine mittlere Primärteilchengröße (Gewichtsmittel) im Bereich von 10 bis < 1000 nm, insbesondere im Bereich von 20 bis 500 nm und insbesondere 50 bis 300 nm, bestimmt durch Lichtstreuung, aufweist (bestimmt z. B. bei 25 °C in einer 0,01 bis 0,1 gew.-%igen wässrigen Dispersion).

Die Herstellung der erfindungsgemäßen Zusammensetzungen wird im Folgenden näher erläutert.

Die erfindungsgemäßen Zusammensetzungen sind in der Regel durch ein Verfahren erhältlich, welches die folgenden Schritte umfasst:
a) Bereitstellung einer wässrigen Dispersion des Astaxanthin-Derivats, die wenigstens ein proteinisches Schutzkolloid enthält, wobei das Astaxanthin-Derivat zu wenigstens 70 Gew.-%, insbesondere wenigstens 80 Gew.-5, besonders bevorzugt wenigstens 90 Gew.-% oder wenigstens 96 Gew.-% aus der Verbindung der Formel I besteht; und
b) Trocknung der wässrigen Dispersion.

Gegenstand der Erfindung ist daher auch ein derartiges Verfahren.

Die Bereitstellung der wässrigen Dispersion des Astaxanthin-Derivats kann in Analogie zu Standardverfahren zur Herstellung wässriger Carotinoid-Dispersionen erfolgen, wie sie beispielsweise aus dem eingangs zitierten Stand der Technik bekannt sind, z. B. aus EP 065193, WO 00/6665, EP 1213013, EP 1219292 und EP 1228705.

In der Regel erfolgt die Bereitstellung der wässrigen Dispersion des Astaxanthin-Derivats dergestalt, dass die Partikelgröße der dispergierten Partikel in der Regel einen gewichtsmittleren bzw. volumenmittleren Partikeldurchmesser von nicht mehr als 5 µm, häufig nicht mehr als 3 µm und insbesondere nicht mehr als 2 µm aufweisen, z. B. im Bereich von 10 nm bis 5 µm, häufig im Bereich von 20 nm bis 3 µm und insbesondere im Bereich von 50 nm bis 2 µm.

Gemäß einer ersten Ausführungsform des erfindungsgemäßen Verfahrens wird man ein festes Astaxanthin-Derivat, das in der Regel kristallin ist, in einer wässrigen Lösung des Schutzkolloids dispergieren und auf die gewünschte Partikelgröße vermahlen. Auf diese Weise werden wässrige Suspensionen des Astaxanthin-Derivats erhalten, worin die Partikel eine mittlere Teilchengröße (Volumenmittel) im Bereich von 0,1 bis 5 µm, insbesondere im Bereich von 0,2 bis 3 µm oder im Bereich von 0,3 bis 2 µm aufweisen (bestimmt durch Lichtstreuung).

Hierzu wird man zunächst eine grobteiligen Suspension des Astaxanthin-Derivats, das vorzugsweise kristallin oder überwiegend kristallin ist (Kristallinitätsgrad > 80 %) in einer wässrigen Lösung des Schutzkolloids bereitstellen. Die wässrige Lösung des Schutzkolloids kann weitere, insbesondere wasserlösliche Bestandteile wie wasserlösliche Antioxidantien und Weichmacher enthalten.

Anschließend erfolgt die Mahlung auf die gewünschte Partikelgröße. Gegebenenfalls kann man während des Mahlens weitere Bestandteile der Formulierung zusetzen. Die Mahlung kann dabei in an sich bekannter Weise z. B. mit einer Kugelmühle erfolgen. Dabei wird je nach verwendetem Mühlentyp so lange gemahlen, bis die Teilchen eine über Fraunhofer Beugung ermittelte mittlere Partikelgröße D[4,3] von 0,1 bis 5 µm, bevorzugt 0,2 bis 3 µm, besonders bevorzugt 0,3 bis 2 µm, ganz besonders bevorzugt 0,3 bis 1,5 µm oder 0,3 bis 1 µm aufweisen. Der Begriff D[4,3] bezeichnet den volumengewichteten mittleren Durchmesser (Volumenmittel, siehe Handbuch zu Malvern Mastersizer S, Malvern Instruments Ltd., UK). Nähere Einzelheiten zur Mahlung und den dafür verwendeten Apparaturen finden sich u. a. in Ullmann's Encyclopedia of Industrial Chemistry, Sixth Edition, 1999, Electronic Release, Size Reduction, Kapitel 3.6.: Wet Grinding sowie in EP-A-0 498 824.

Gemäß einer zweiten Ausführungsform des erfindungsgemäßen Verfahrens wird man die Dispersion des Astaxanthin-Derivats dadurch bereitstellen, dass man eine Lösung des Astaxanthin-Derivats in einem geeigneten organischen Lösungsmittel oder in einer Mischung aus Wasser und einem geeigneten organischen Lösungsmittel in einer wässrigen Lösung des Schutzkolloids einbringt und anschließend das Lösungsmittel entfernt. Beim Einbringen der Lösung des Astaxanthin-Derivats in die wässrige Lösung des Schutzkolloids kommt es zu einer Fällung des Astaxanthin-Derivats und damit zur Ausbildung von Nanopartikeln des Astaxanthin-Derivats, worin das Astaxanthin-Derivat überwiegend oder vollständig amorph vorliegt. Auf diese Weise werden wässrige Dispersionen des Astaxanthin-Derivats erhalten, worin die Partikel eine mittlere Teilchengröße (Volumenmittel) im Bereich von 10 bis < 1000 nm, insbesondere im Bereich von 20 bis 500 nm und speziell im Bereich von 50 bis 300 nm, bestimmt durch Lichtstreuung, aufweisen.

Die gemäß der zweiten Ausführungsform des erfindungsgemäßen Verfahrens zur Herstellung der Suspension verwendeten organischen Lösungsmittel sind in der Regel mit Wasser mischbar. Unter einem mit Wasser mischbaren organischen Lösungsmittel versteht man solche Lösungsmittel, die bei 25 °C zu wenigstens 10 Vol.-%, insbesondere zu wenigstens 50 Vol.-% oder vollständig mit Wasser mischbar sind. Bevorzug ist das in Schritt a) der zweiten Ausführungsform eingesetzte organische Lösungsmittel mit Wasser mischbar oder eine Mischung aus Wasser und einem mit Wasser mischbaren organischen Lösungsmittel. Bevorzugt sind solche organischen Lösungsmittel, die aus Kohlenstoff, Wasserstoff und Sauerstoff aufgebaut sind und mit Wasser mischbar sind. Vorzugsweise weist das organische Lösungsmittel bei Normaldruck einen Siedepunkt unterhalb 200 °C auf.

Bevorzugte organische, mit Wasser mischbare Lösungsmittel sind aus den Gruppen der C₁-C₄-Alkanole, C₂-C₄-Alkandiole, C₃-C₄-Ketone, Etheralkohole mit 3 bis 6 C-Atomen und cyclischen Ethern mit 4 bis 5 C-Atomen ausgewählt. Beispiele für bevorzugte, mit Wasser mischbare Lösungsmittel sind Methanol, Ethanol, n-Propanol, Isopropanol, Propandiol, Ethylenglykolmethylether, 1,2-Butandiol-1-methylether, 1,2-Propandiol-1-n-propylether, Tetrahydrofuran oder Aceton.

Vorzugsweise wird man bei der Herstellung gemäß der zweiten Ausführungsform so vorgehen, dass man die Lösung des Astaxanthin-Derivats in dem organischen Lösungsmittel oder einer Mischung aus organischem Lösungsmittel und Wasser, die eine Temperatur von wenigstens 30 °C, insbesondere wenigstens 50 °C, besonders bevorzugt wenigstens 100 °C, z. B. 30 bis 200 °C, insbesondere 50 bis 190 °C oder 80 bis 180 °C aufweist, in eine wässrige Lösung des proteinischen Schutzkolloids einträgt und dadurch eine Fällung oder Emulgierung des Astaxanthin-Derivats bewirkt. Vorzugsweise weist die wässrige Lösung des proteinischen Schutzkolloids eine Temperatur unterhalb der Temperatur der Lösung des Astaxanthin-Derivats auf und wird vorzugsweise eine Temperatur von 50 °C nicht überschreiten. Vorzugsweise wird die Temperatur und Menge der wässrigen Lösung des proteinischen Schutzkolloids so gewählt, dass eine Mischungstemperatur unterhalb 100 °C, z. B. im Bereich von 20 bis 80 °C, resultiert.

Vorzugsweise wählt man die Bedingungen des Lösens so, dass die zum Lösen benötigte Zeit nicht mehr als 20 sec., insbesondere nicht mehr als 10 sec. und speziell nicht mehr als 2 sec. beträgt, z. B. 0,1 bis 20 sec. oder 0,1 bis 1 sec. Vorzugsweise erfolgt das Lösen unter Druck, z. B. bei einem Druck im Bereich von 10 bis 100 bar und insbesondere im Bereich von 20 bis 80 bar.

Die Konzentration an Astaxanthin-Derivat in der Lösung wird in der Regel im Bereich von 0,1 bis 10 Gew.-% liegen. Die Lösung des Astaxanthin-Derivats kann gegebenenfalls weitere Bestandteile der erfindungsgemäßen Zusammensetzung enthalten, die in dem Lösungsmittel bzw. in dem Lösungsmittel/Wassergemisch löslich sind. Hierzu zählen insbesondere die vorgenannten Antioxidantien, Emulgatoren und Öle.

Die Konzentration an proteinischem Schutzkolloid in der wässrigen Lösung wird in der Regel im Bereich von 0,1 bis 10 Gew.-% liegen. Die Lösung des proteinischen Schutzkolloids kann gegebenenfalls weitere Bestandteile der erfindungsgemäßen Zusammensetzung enthalten, die in Wasser löslich oder suspendierbar sind. Hierzu zählen insbesondere die vorgenannten Weichmacher und Konservierungsmittel.

Die relativen Mengen an Lösung des Astaxanthin-Derivats und wässriger Lösung des proteinischen Schutzkolloids werden in der Regel so gewählt, dass die Mengenanteile der darin enthaltenen Bestandteile den Mengenanteilen dieser Bestandteile in erfindungsgemäßen Zusammensetzungen im Wesentlichen entsprechen.

Vorzugsweise führ man das Lösen und Einbringen in die Lösung des proteinischen Schutzkolloids in Analogie zu der in EP 65193 beschriebenen Vorgehensweise durch. Hinsichtlich einer detaillierten Apparatebeschreibung wird auf die EP 65193 und die Beispiele dieser Anmeldung Bezug genommen.

Häufig wird man so vorgehen, dass man das Astaxanthin-Derivat, gegebenenfalls zusammen mit dem Antioxidationsmittel, gegebenenfalls dem Emulgator und gegebenenfalls dem Öl in dem organischen Lösungsmittel oder einer Mischung aus organischem Lösungsmittel und Wasser unter den zuvor genannten Bedingungen in ein oder mehreren Stufen löst, vorzugsweise bei erhöhter Temperatur und erhöhtem Druck, löst und möglichst rasch in die wässrige Lösung des Schutzkolloids, die weitere Bestandteile wie Konservierungsmittel und Weichmacher enthalten kann, einträgt, wobei das Eintragen möglichst rasch erfolgt und geeignete Maßnahmen zur Homogenisierung der wässrigen Lösung des Schutzkolloids und der zugeführten Lösung des Astaxanthin-Derivats vorgesehen sind.

Insbesondere wird man so vorgehen, dass man das Astaxanthin-Derivat in dem organischen Lösungsmittel oder einer Mischung aus Wasser und organischem Lösungsmittel, gegebenenfalls zusammen mit den weiteren Bestandteilen der Lösung vermischt und kontinuierlich, gegebenenfalls unter Zuführung von weiterem Lösungsmittel, erhitzt, wobei man einen Volumenstrom der Lösung des Astaxanthin-Derivats erzeugt, den man mit einem Volumenstrom der Lösung Schutzkolloids in einer geeigneten Mischvorrichtung, beispielsweise einem statischen oder dynamischen Mischer, vermischt, wobei man die wässrige Suspension des Astaxanthin-Derivats, enthaltend das proteinische Schutzkolloid, erhält. Die Volumenströme werden dabei so gewählt, dass die Mengenanteile der darin enthaltenen Bestandteile den Mengenanteilen dieser Bestandteile in erfindungsgemäßen Zusammensetzungen im Wesentlichen entsprechen.

Die auf diese Weise erhaltenen wässrigen Dispersionen des Astaxanthin-Derivats enthalten typischer 0,01 bis 5 Gew.-% des Astaxanthin-Derivats und 0,1 bis 10 Gew.-% des proteinischen Schutzkolloids.

Die so erhaltene Dispersion kann anschließend einer üblichen Trocknung zugeführt werden. Die Trocknung kann in einer oder mehreren Stufen erfolgen. Vorzugsweise wird man zunächst die in Schritt a) erhaltene Dispersion aufkonzentrieren, vorzugsweise auf einen Gehalt an Astaxanthin-Derivat im Bereich von 1 bis 20 Gew.-%, und anschließend den eigentlichen Trocknungsschritt durchführen.

Das Aufkonzentrieren kann in hierzu üblichen Vorrichtungen, beispielsweise Verdampferanordnungen wie Dünnfilmverdampfern und dergleichen erfolgen.

Die Trocknung kann nach üblichen Verfahren zur Herstellung von Pulvern aus wässrigen Dispersionen erfolgen. Zu nennen sind beispielsweise wie Sprühtrocknung, Sprühkühlung, Gefriertrocknung, Sprühformulierung oder Sprühgranulierung, wobei Sprühtrocknung und Sprühformulierung, d. h. Sprühtrocknung in Gegenwart eines im Wirbelbett fluidierten inerten Pulvers bevorzugt sind. Gegebenenfalls kann die Trocknung in Gegenwart eines Überzugsmaterials erfolgen. Als Überzugsmittel eignen sich u. a. Maisstärke, Kieselsäure oder auch Tricalciumphosphat.

Die erfindungsgemäßen Pulver lassen sich direkt in für Nahrungsmittel geeigneten Ölen suspendieren. Beim Suspendieren tritt überraschenderweise das Astaxanthin-Derivat, bzw. die Hauptmenge des im Pulver enthaltenen Astaxanthin-Derivats, in die Ölphase über, so dass Lösungen des Astaxanthin-Derivats in dem Öl erhalten werden. Anders als in den Astaxanthin-Trockenpulvern des Standes der Technik muss hierfür das Pulver nicht zuvor wieder in eine wässrige Suspension überführt werden. Dementsprechend eignen sich die erfindungsgemäßen Zusammensetzungen in besonderer Weise zur Herstellung von Futtermitteln. Darüber hinaus hat sich gezeigt, dass bei Einarbeitung einer wässrigen Dispersion der erfindungsgemäßen Zusammensetzung, im Folgenden auch als wässriges Redispergat bezeichnet, in ein für Nahrungsmittel geeignetes Öl, der Übertritt des Astaxanthin-Derivats in die Ölphase sehr viel rascher und vollständiger erfolgt und auch bei niedrigeren Temperaturen stattfindet als bei vergleichbaren pulverförmigen Zusammensetzungen des Astaxanthins selber.

Dementsprechend betrifft die vorliegende Erfindung auch die Verwendung der erfindungsgemäßen Zusammensetzungen zur Herstellung von Lösungen des Astaxanthin-Derivats in für Nahrungsmittel geeigneten Ölen sowie die Verwendung der Zusammensetzungen zur Herstellung von Futtermittelzubereitungen, insbesondere von festen Futtermittelzubereitungen, speziell Futtermittelzubereitungen in Form von Pellets.

Dementsprechend betrifft ein weiterer Gegenstand der Erfindung ein Verfahren zur Herstellung von Futtermittelzubereitungen, insbesondere von festen Futtermittelzubereitungen, speziell von Pellets, umfassend:
α) Bereitstellung einer erfindungsgemäßen pulverförmigen Zusammensetzung wie hier und in den Ansprüchen beschrieben;
β) Einarbeiten der Zusammensetzung in ein für Nahrungsmittel geeignetes, flüssiges Öl unter Erhalt einer verdünnten flüssigen Lösung des Astaxanthin-Derivats in dem flüssigen Öl; und
γ) Vermischen der in Schritt β) erhaltenen flüssigen Lösung mit den Bestandteilen der Futtermittelzubereitung oder Imprägnieren einer festen Futtermittelzubereitung mit der in Schritt β) erhaltenen flüssigen Lösung.

Das in Schritt β) eingesetzte Öl kann synthetischer, mineralischer, pflanzlicher oder tierischer Herkunft sein. Beispiel sind Pflanzenöle wie Sojabohnenöl, Sonnenblumenöl, Maiskeimöl, Leinsamenöl, Rapsöl, Distelöl, Weizenkeimöl, Reisöl, Kokosöl, Mandelöl, Aprikosenkernöl, Palmöl, Palmkernöl, Avocadoöl, Jojobaöl, Haselnussöl, Walnussöl, Erdnussöl, Pistazienöl, Triglyceride mittelkettiger (= C₈-C₁₀) pflanzlicher Fettsäuren (sog. MCT-Öle) und PUFA-Öle (PUFA = mehrfach ungesättigte Fettsäuren (polyunsaturated fatty acids) wie Eikosapentaensäure (EPA), Docosahexaensäure (DHA) und α-Linolensäure), weiterhin semisynthetische Triglyceride, z. B. Caprylsäure/Caprinsäure-Tricyceride wie die Miglyol-Typen, weiterhin Paraffinöl, flüssige hydrierte Polyisobutene, Squalan, Squalen, weiterhin tierische Öle und Fette wie Fischöle einschließlich Makrelen-, Sprotten-, Thunfisch-, Heilbutt-, Kabeljau- und Lachsöl.

Bevorzugt sind pflanzliche Öle und Öle tierischen Ursprungs, die bei 40 °C flüssig sind, insbesondere Pflanzenöle wie Sojabohnenöl, Sonnenblumenöl, Distelöl, Maiskeimöl, Olivenöl, Leinsamenöl, Rapsöl, Reisöl, Kokosöl, Erdnussöl, Palmöl, Palmkernöl, PUFA-Öle, MCT-Öle, weiterhin Fischöle, sowie Mischungen dieser Öle.

In einer bevorzugten Ausführungsform der Erfindung umfasst das in Schritt β) eingesetzte Öl zu wenigstens 50 Gew.-% und insbesondere zu wenigstens 80 Gew.-%, bezogen auf die Gesamtmenge des in Schritt β) eingesetzten essbaren Öls, wenigstens ein unter Maisöl, Sonnenblumenöl, Sojabohnenöl und Fischöl ausgewähltes essbares Öl.

Bei den in Schritt β) eingesetzten Ölen kann es sich um Raffinatöle oder Rohöle handeln, die noch ursprungsspezifische Verunreinigungen wie Proteine, Phosphat, (Erd)alkalimetallsalze und dergleichen in üblichen Mengen enthalten. Die in Schritt β) eingesetzten Öle können auch geringe Mengen an emulgiertem oder gelöstem Wasser, vorzugsweise jedoch nicht mehr als 10 Gew.-%, insbesondere nicht mehr als 1 Gew.-%, bezogen auf die Gesamtmenge des in Schritt b) eingesetzten Öls, enthalten.

Das Einarbeiten der pulverförmigen Zusammensetzung in das für Nahrungsmittel geeignete Öl erfolgt typischerweise bei Temperaturen unterhalb 100 °C, vorzugsweise bei Temperaturen von nicht mehr als 95 °C und speziell nicht mehr als 90 °C und vorzugsweise bei Temperaturen im Bereich von 20 bis < 100 °C, insbesondere im Bereich von 25 bis 95 °C und speziell im Bereich von 30 bis 90 °C.

Vorzugsweise wird man das Öl, in welches man die pulverförmige Zusammensetzung des Astaxanthin-Derivats einarbeitet, erwärmen. In der Regel liegt die Temperatur des erwärmten Öls vorzugsweise unterhalb 100 °C und beträgt insbesondere nicht mehr als 95 °C und speziell nicht mehr als 90 °C, und liegt vorzugsweise im Bereich von 25 bis < 100 °C, insbesondere im Bereich von 30 bis 95 °C und speziell im Bereich von 35 bis 90 °C.

Typischerweise wird man die relativen Mengen an Öl und erfindungsgemäßer Zusammensetzung so wählen, dass der Gehalt an Astaxanthin-Derivat in der resultierenden Lösung im Bereich von 10 ppm (= 0,001 Gew.-%) bis etwa 1 Gew.-%, häufig im Bereich von 20 ppm bis 0,5 Gew.-%, insbesondere im Bereich von 50 ppm bis 0,2 Gew.-%, bezogen auf die Gesamtmenge an Öl plus Astaxanthin-Derivat liegt.

Beispielsweise kann man so vorgehen, dass man ein für Nahrungsmittel geeignetes Öl, das vorzugsweise einen Schmelzpunkt von nicht mehr als 40 °C aufweist, in einem geeigneten Gefäß vorlegt, gegebenenfalls auf die gewünschte Temperatur vorwärmt und hierzu die erfindungsgemäße Zusammensetzung unter Durchmischen zugibt, wobei die Zugabe in einer Portion, in mehreren Portionen oder kontinuierlich erfolgen kann. Die zugegebene erfindungsgemäße Zusammensetzung kann auf die gewünschte Temperatur vorgewärmt werden, was jedoch grundsätzlich nicht erforderlich ist.

Die zum Erreichen des Lösens erforderliche Zeit hängt naturgemäß von der Temperatur, dem gewählten Öl und den apparativen Gegebenheiten ab und liegt typischerweise im Bereich von 1 min. bis 120 min. Der Fachmann kann die erforderliche Zeit durch Routineexperimente ermitteln.

Im Anschluss an das Lösen der erfindungsgemäßen Zusammensetzung kann man die erhaltene ölige Lösung, sofern erforderlich, abkühlen, z. B. mittels geeigneter Wärmetauscher oder durch Verdünnen mit einem kalten essbaren Öl, das zu dem zum Lösen in Schritt β) verwendeten Öl gleich oder verschieden sein kann.

Auf diese Weise erhält man stabile Lösungen von Astaxanthin-Derivaten in für Nahrungsmittel geeigneten Ölen. Diese Lösungen zeichnen sich dadurch aus, dass das in ihnen enthaltene Astaxanthin-Derivat einen hohen Anteil an all-trans-Isomeren aufweist, der in der Regel oberhalb 80 %, insbesondere oberhalb 90 % liegt.

Die so erhaltenen Lösungen enthalten neben dem Astaxanthin-Derivat die in der Zusammensetzung enthaltenen Substanzen, die sich nicht notwendigerweise lösen müssen, sowie ggf. weitere Zusätze wie Oxidationsstabilisatoren und lipophile Dispergiermittel.

Es versteht sich von selber, dass die in Schritt β) erhaltenen Lösungen geringe Mengen an Wasser aus dem zu ihrer Herstellung eingesetzten, für Nahrungsmittel geeigneten Öl enthalten können. Häufig jedoch beträgt der Wasseranteil nicht mehr als 10 Gew.-%, z. B. 0,1 bis 10 Gew.-%, häufig 0,5 bis 8 Gew.-%, und in einer speziellen Ausführungsform der Erfindung nicht mehr als 0,5 Gew.-%, bezogen auf das in der Lösung enthaltene Öl.

Die nach dem erfindungsgemäßen Verfahren erhältlichen Lösungen des Astaxanthin-Derivats sind lagerstabil und können vor ihrer weiteren Verwendung über längere Zeiträume aufbewahrt werden, ohne dass es in nennenswertem Umfang zu einem Aktivitätsverlust, z. B. durch Isomerisierung und/oder oxidativen Abbau, kommt. Insbesondere zeichnen sie sich durch einen hohen Anteil an all-trans-Isomeren des eingesetzten Astaxanthin-Derivats aus und durch einen vergleichsweise geringen Anteil an Abbauprodukten des Astaxanthin-Derivats.

Das erfindungsgemäße Verfahren wird häufig direkt in die Prozesse zur Weiterverarbeitung der Lösungen des Astaxanthin-Derivats integriert werden.

Die nach dem erfindungsgemäßen Verfahren erhältlichen Lösungen des Astaxanthin-Derivats eignen sich in vorteilhafter Weise als Zusatz zu Tierfuttermitteln, Nahrungsmitteln für die Humanernährung, Nahrungsergänzungsmitteln, pharmazeutischen Mitteln oder kosmetischen Mitteln. Bevorzugt lassen sich die Lösungen als Futtermittelzusatz in der Tieremährung einsetzen, beispielsweise bei der Futtermittelherstellung durch Einmischen in einen Futtermittelteig vor oder während der Extrusion oder durch Auftragen bzw. Aufsprühen auf Futtermittelpellets. Die Anwendung als Futtermittelzusatz erfolgt insbesondere durch direktes Aufsprühen der erfindungsgemäßen Formulierungen, beispielsweise als so genannte "post-pelleting liquid application". Vorzugsweise belädt man die Futtermittelpellets mit den Formulierungen unter vermindertem Druck.

Dementsprechend betrifft die vorliegende Erfindung auch die Herstellung von Futtermitteln, wie Tierfuttermittel, Nahrungsmittel für die Humanernährung, Nahrungsergänzungsmittel, sowie die Herstellung pharmazeutischer Mittel oder kosmetischer Mittel unter Verwendung der erfindungsgemäß hergestellten Lösungen des Astaxanthin-Derivats. Diese Mittel enthalten neben den für diese Mittel üblichen Bestandteilen das in Schritt β) eingesetzte Öl, das proteinische Schutzkolloid, das Astaxanthin-Derivat und gegebenenfalls weitere Bestandteile der erfindungsgemäßen Zusammensetzung.

Das Verfahren umfasst die folgenden Schritte:
α) Bereitstellung einer erfindungsgemäßen pulverförmigen Zusammensetzung;
β) Einarbeiten der pulverförmigen Zusammensetzung in ein für Nahrungsmittel geeignetes, flüssiges Öl unter Erhalt einer verdünnten flüssigen Lösung des Astaxanthin-Derivats in dem flüssigen Öl; und
γ) Vermischen der in Schritt β) erhaltenen flüssigen Lösung mit den Bestandteilen der Futtermittelzubereitung oder Imprägnieren einer festen Futtermittelzubereitung mit der in Schritt β) erhaltenen flüssigen Lösung.

Bevorzugte Ausführungsformen betreffen Tierfuttermittel, insbesondere Fischfuttermittel, die das bei 30 °C flüssige Öl, das feste Verdünnungsmittel und das Astaxanthin-Derivat umfassen. Derartige Mittel enthalten das in der Lösung enthaltene Astaxanthin-Derivat typischerweise in einer Menge von 10 bis 100 ppm, bezogen auf das Gesamtgewicht des Mittels, wobei das Astaxanthin-Derivat in der Regel zu mehr als 70 %, insbesondere zu wenigstens 80 % und speziell zu wenigstens 90 % eine all-trans-Konfiguration aufweist.

Typische Bestandteile in Futtermitteln sind Kohlehydrat-Quellen, insbesondere Getreidemehle wie Weizen- oder Maismehl, Sojabohnenmehl, aber auch Zucker und Zuckeralkohole, weiterhin proteinhaltige Bestandteile wie Sojakonzentrat, Fischmehl, Glutene wie Mais- oder Weizengluten, Öle und Fette, z. B. die zuvor genannten essbaren Öle, aber auch andere essbare Fette pflanzlichen oder tierischen Ursprungs, weiterhin Nutrazeutika wie freie Aminosäuren, deren Salze, Vitamine und Spurenelemente, sowie gegebenenfalls Verarbeitungshilfsmittel, z. B. Gleitmittel, Antiblockmittel, inerte Füllstoffe und dergleichen, und gegebenenfalls Konservierungsmittel. Typische Fischfutterzusammensetzungen enthalten z. B. Getreidemehl in einer Menge von beispielsweise 3 bis 20 Gew.-%, Gluten, z. B. in einer Menge von 1 bis 30 Gew.-%, ein oder mehrere Proteinquellen, z. B. Sojakonzentrat und/oder Fischmehl, z. B. in einer Gesamtmenge von 10 bis 50 Gew.-%, Fette und/oder Öle in einer Menge von beispielsweise 10 bis 50 Gew.-%, gegebenenfalls ein oder mehrere Vitamine in einer Gesamtmenge von beispielsweise 0,1 bis 2 Gew.-% und gegebenenfalls Aminosäuren in einer Menge von beispielsweise 0,1 bis 5 Gew.-%, jeweils bezogen auf die Gesamtmenge der Futtermittelbestandteile.

Eine spezielle Ausführungsform dieser Mittel betrifft Futtermittelpellets, speziell Futtermittelpellets für Fischfutter, die mit der erfindungsgemäß erhältlichen Lösung des Astaxanthin-Derivats beladen sind. Derartige Pellets enthalten das in der Lösung enthaltene Astaxanthin-Derivat typischerweise in einer Menge von 10 bis 100 ppm, bezogen auf das Gesamtgewicht des Futtermittels. Die Herstellung solcher Pellets erfolgt in der Regel durch Besprühen konventioneller Pellets mit einer erfindungsgemäß erhältlichen Lösung des Astaxanthin-Derivats, vorzugsweise unter vermindertem Druck, wobei das Besprühen kontinuierlich oder vorzugsweise diskontinuierlich erfolgen kann. Beispielsweise kann man die konventionellen Pellets in einem geeigneten Gefäß vorlegen, das Gefäß evakuieren und dann Öl unter Durchmischen der Pellets aufsprühen und anschließend belüften. Auf diese Weise erreicht man ein gleichmäßiges Eindringen der erfindungsgemäßen öligen Zusammensetzung in die Pellets. Gegebenenfalls kann man erneut Vakuum anlegen und erneut die Lösung des Astaxanthin-Derivats oder ein für Nahrungsmittel geeignetes, flüssiges Öl in der zuvor beschriebenen Weise aufsprühen. Auf diese Weise erhält man Pellets, die im Kern das Öl und das Astaxanthin-Derivat enthalten.

## Patentansprüche

1. Pulverförmige Zusammensetzung eines Astaxanthin-Derivats, bestehend aus:
i) einem Astaxanthin-Derivat in einer Menge von 1 bis 50 Gew.-%, bezogen auf das Gesamtgewicht der Zusammensetzung,
ii) wenigstens einem proteinischen Schutzkolloid in einer Menge von wenigstens 10 bis 70 Gew.-%, bezogen auf das Gesamtgewicht der Zusammensetzung, und
iii) gegebenenfalls einem oder mehreren, für Nahrungsmittel geeigneten Zusatzstoffen in einer Menge von bis zu 89 Gew.-%, bezogen auf das Gesamtgewicht der Zusammensetzung,
wobei das Astaxanthin-Derivat zu wenigstens 70 Gew.-% aus wenigstens einer Verbindung der Formel I 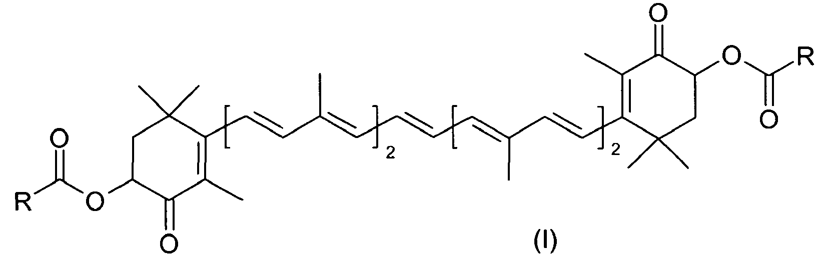 besteht, worin R für einen Rest der Formel 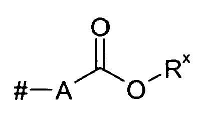 steht, worin # die Anknüpfung an die Carbonylgruppe bedeutet, A für -CH₂CH₂-oder für -CH=CH- steht und R^{x} für C₁-C₄ Alkyl steht.

2. Zusammensetzung nach Anspruch 1, wobei in Formel I der Rest R^{x} Methyl oder Ethyl bedeutet und A für -CH₂CH₂- steht.

3. Zusammensetzung nach einem der vorhergehenden Ansprüche, worin das Schutzkolloid ausgewählt ist unter Casein, Caseinaten, Molkeproteinen, Prolaminen und deren Mischungen.

4. Zusammensetzung nach Anspruch 3, worin das Schutzkolloid Casein und/oder Caseinat umfasst.

5. Zusammensetzung nach einem der vorhergehenden Ansprüche, enthaltend weiterhin wenigstens einen weiteren für Nahrungsmittel geeigneten Zusatz, der unter Oxidationsstabilisatoren, Emulgatoren, Konservierungsmitteln, Weichmachern, Filmbildnern, Füllstoffen, Ölen, Fließhilfsmitteln und Puderungsmitteln ausgewählt ist.

6. Zusammensetzung nach Anspruch 5, enthaltend wenigstens einen Weichmacher, der unter Mono-, Di- und Oligosacchariden und Zuckeralkoholen und deren Gemischen ausgewählt ist.

7. Zusammensetzung nach einem der vorhergehenden Ansprüche, worin das Astaxanthin-Derivat zu wenigstens 70 % in der all-trans-Form vorliegt.

8. Zusammensetzung nach einem der vorhergehenden Ansprüche, wobei eine wässrige Dispersion der Pulverteilchen in Wasser eine mittlere Primärteilchengröße (Gewichtsmittel) im Bereich von 10 bis 2000 nm, bestimmt durch Lichtstreuung, aufweist.

9. Zusammensetzung nach einem der vorhergehenden Ansprüche, erhältlich durch ein Verfahren, welches die folgenden Schritte umfasst:
a) Bereitstellung einer wässrigen Dispersion des Astaxanthin-Derivats, die wenigstens ein proteinisches Schutzkolloid enthält, wobei das Astaxanthin-Derivat zu wenigstens 70 Gew.-% aus der Verbindung der Formel I besteht; und
b) Trocknung der wässrigen Dispersion.

10. Zusammensetzung nach Anspruch 9, wobei die wässrige Dispersion des Astaxanthin-Derivats erhältlich ist durch ein Verfahren, umfassend die folgenden Schritte:
a1) Lösen des Astaxanthin-Derivats in einem organischen Lösungsmittel, das aus Kohlenstoff, Wasserstoff und Sauerstoff besteht,
a2) Vermischen der in Schritt a1) erhaltenen Lösung mit einer wässrigen Zusammensetzung, die wenigstens ein proteinisches Schutzkolloid in gelöster Form enthält.

11. Verfahren zur Herstellung einer Zusammensetzung nach einem der vorhergehenden Ansprüche, umfassend die folgenden Schritte:
a) Bereitstellung einer wässrigen Dispersion des Astaxanthin-Derivats, die wenigstens ein proteinisches Schutzkolloid enthält, wobei das Astaxanthin-Derivat zu wenigstens 70 Gew.-% aus der Verbindung der Formel I bssteht; und
b) Trocknung der wässrigen Dispersion.

12. Verfahren nach Anspruch 11, wobei die Bereitstellung der wässrigen Dispersion des Astaxanthin-Derivats die folgenden Schritte umfasst:
a1) Lösen des Astaxanthin-Derivats in einem organischen Lösungsmittel, das aus Kohlenstoff, Wasserstoff und Sauerstoff besteht,
a2) Vermischen der in Schritt a1) erhaltenen Lösung mit einer wässrigen Zusammensetzung, die wenigstens ein proteinisches Schutzkolloid in gelöster Form enthält.

13. Verfahren nach Anspruch 11, wobei die Bereitstellung der wässrigen Dispersion in Schritt i) das Vermischen von festem Astaxanthin-Derivat mit einer wässrigen Lösung des Schutzkolloids und das Vermahlen der Mischung umfasst.

14. Verfahren nach einem der Ansprüche 11 bis 13, wobei die Trocknung der wässrigen Dispersion durch Sprühtrocknung oder Sprühformulierung erfolgt.

15. Verwendung einer Zusammensetzung nach einem der Ansprüche 1 bis 10 zur Herstellung von Futtermittelzubereitungen, insbesondere festen Futtermittelzubereitungen, speziell Futtermittelzubereitungen in Form von Pellets.

16. Verwendung einer Zusammensetzung nach einem der Ansprüche 1 bis 10 zur Herstellung von Lösungen des Astaxanthin-Derivats in einem für Nahrungsmittel geeigneten Öl.

17. Verfahren zur Herstellung von Futtermittelzubereitungen, insbesondere von festen Futtermittelzubereitungen, speziell von Pellets, umfassend:
α) Bereitstellung einer pulverförmigen Zusammensetzung gemäß einem der Ansprüche 1 bis 10;
β) Einarbeiten der pulverförmigen Zusammensetzung in ein für Nahrungsmittel geeignetes, flüssiges Öl unter Erhalt einer verdünnten flüssigen Lösung des Astaxanthin-Derivats in dem flüssigen Öl; und
γ) Vermischen der in Schritt β) erhaltenen flüssigen Lösung mit den Bestandteilen der Futtermittelzubereitung oder Imprägnieren einer festen Futtermittelzubereitung mit der in Schritt β) erhaltenen flüssigen Lösung.

## Claims

1. A pulverulent composition of an astaxanthin derivative comprising:
i)an astaxanthin derivative in an amount of 1 to 50% by weight, based on the total weight of the composition,
ii) at least one proteinaceous protective colloid in an amount of at least 10 to 70% by weight, based on the total weight of the composition, and
iii) optionally one or more additives suitable for nourishments in an amount of up to 89% by weight, based on the total weight of the composition,
wherein the astaxanthin derivative comprises at least 70% by weight of at least one compound of the formula I 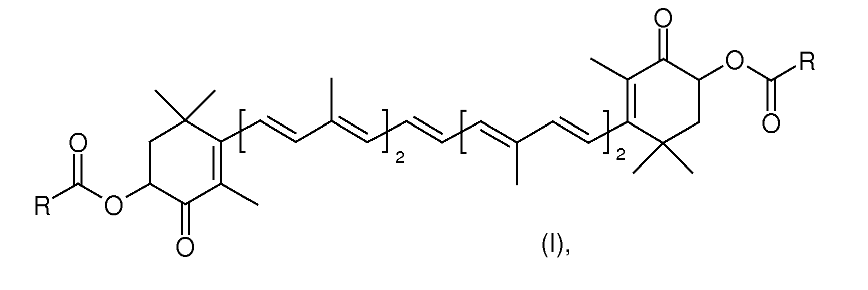 where R is a radical of the formula 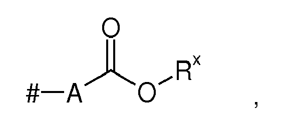 where # denotes the linkage to the carbonyl group, A is -CH₂CH₂- or -CH=CH- and R^{x} is C₁-C₄ alkyl.

2. The composition according to claim 1, wherein, in formula I, the radical R^{x} is methyl or ethyl and A is -CH₂CH₂-.

3. The composition according to any one of the preceding claims, where the protective colloid is selected from casein, caseinates, whey proteins, prolamins and mixtures thereof.

4. The composition according to claim 3, where the protective colloid comprises casein and/or caseinate.

5. The composition according to any one of the preceding claims, further comprising at least one further additive suitable for nourishments which is selected from oxidation stabilizers, emulsifiers, preservatives, plasticizers, film-forming agents, fillers, oils, free-flowing aids and powdering agents.

6. The composition according to claim 5, comprising at least one plasticizer which is selected from mono-, di- and oligosaccharides and sugar alcohols and mixtures thereof.

7. The composition according to any one of the preceding claims, where the astaxanthin derivative is present to at least 70% in the all-trans form.

8. The composition according to any one of the preceding claims, wherein an aqueous dispersion of the powder particles in water has a median primary particle size (weight average) in the range from 10 to 2000 nm, determined by light scattering.

9. The composition according to any one of the preceding claims, obtainable by a process which comprises the following steps:
a) providing an aqueous dispersion of the astaxanthin derivative which comprises at least one proteinaceous protective colloid, wherein the astaxanthin derivative comprises at least 70% by weight of the compound of the formula I; and
b)drying the aqueous dispersion.

10. The composition according to claim 9, wherein the aqueous dispersion of the astaxanthin derivative is obtainable by a process which comprises the following steps:
a1) dissolving the astaxanthin derivative in an organic solvent which comprises carbon, hydrogen and oxygen,
a2) mixing the solution obtained in step a1) with an aqueous composition which comprises at least one proteinaceous protective colloid in dissolved form.

11. A process for producing a composition according to any one of the preceding claims, which comprises the following steps:
a)providing an aqueous dispersion of the astaxanthin derivative which comprises at least one proteinaceous protective colloid, wherein the astaxanthin derivative comprises at least 70% by weight of the compound of the formula I; and
b)drying the aqueous dispersion.

12. The process according to claim 11, wherein providing the aqueous dispersion of the astaxanthin derivative comprises the following steps:
a1) dissolving the astaxanthin derivative in an organic solvent which comprises carbon, hydrogen and oxygen,
a2) mixing the solution obtained in step a1) with an aqueous composition which comprises at least one proteinaceous protective colloid in dissolved form.

13. The process according to claim 11, wherein providing the aqueous dispersion in step i) comprises mixing solid astaxanthin derivative with an aqueous solution of the protective colloid and milling the mixture.

14. The process according to any one of claims 11 to 13, wherein drying of the aqueous dispersion is performed by spray drying or spray formulating.

15. The use of a composition according to any one of claims 1 to 10 for producing feed preparations, in particular solid feed preparations, especially feed preparations in the form of pellets.

16. The use of a composition according to any one of claims 1 to 10 for producing solutions of the astaxanthin derivative in an oil suitable for nourishments.

17. A process for producing feed preparations, in particular solid feed preparations, especially pellets, which comprises:
a)providing a pulverulent composition according to any one of claims 1 to 10;
β) incorporating the pulverulent composition into a liquid oil suitable for nourishments, obtaining a dilute liquid solution of the astaxanthin derivative in the liquid oil; and
γ) mixing the liquid solution obtained in step
β) with the components of the feed preparation, or impregnating a solid feed preparation with the liquid solution obtained in step β).

## Revendications

1. Composition sous forme de poudre d'un dérivé d'astaxanthine, constituée par :
i) un dérivé d'astaxanthine en une quantité de 1 à 50% en poids, par rapport au poids total de la composition,
ii) au moins un colloïde de protection protéinique en une quantité d'au moins 10 à 70% en poids, par rapport au poids total de la composition, et
iii) le cas échéant un ou plusieurs additifs appropriés pour aliments, en une quantité allant jusqu'à 89% en poids, par rapport au poids total de la composition,
le dérivé d'astaxanthine étant constitué, à raison d'au moins 70% en poids, par au moins un composé de formule I 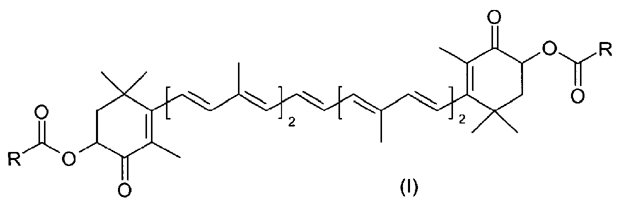 R représentant un radical de formule 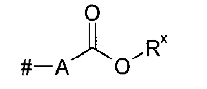 où # signifie la liaison au groupe carbonyle, A représente -CH₂CH₂- ou -CH=CH- et R^{x} représente C₁-C₄-alkyle.

2. Composition selon la revendication 1, où, dans la formule I, le radical R^{x} signifie méthyle ou éthyle et A représente -CH₂CH₂-.

3. Composition selon l'une quelconque des revendications précédentes, le colloïde de protection étant choisi parmi la caséine, les caséinates, les protéines de lactosérum, les prolamines et leurs mélanges.

4. Composition selon la revendication 3, le colloïde de protection comprenant de la caséine et/ou du caséinate.

5. Composition selon l'une quelconque des revendications précédentes, contenant en outre au moins un autre additif approprié pour aliments, qui est choisi parmi les stabilisateurs de l'oxydation, les émulsifiants, les conservateurs, les plastifiants, les agents filmogènes, les charges, les huiles, les fluidifiants et les agents de poudrage.

6. Composition selon la revendication 5, contenant au moins un plastifiant choisi parmi les monosaccharides, les disaccharides, les oligosaccharides et les alcools de sucre et leurs mélanges.

7. Composition selon l'une quelconque des revendications précédentes, le dérivé d'astaxanthine se trouvant à raison d'au moins 70% dans la forme all-trans.

8. Composition selon l'une quelconque des revendications précédentes, une dispersion aqueuse des particules de poudre dans l'eau présentant une grosseur moyenne des particules primaires (moyenne pondérale) dans la plage de 10 à 2000 nm, déterminée par diffusion de la lumière.

9. Composition selon l'une quelconque des revendications précédentes, pouvant être obtenue par un procédé comprenant les étapes suivantes :
a) préparation d'une dispersion aqueuse du dérivé d'astaxanthine, qui contient au moins un colloïde de protection protéinique, le dérivé d'astaxanthine étant constitué à raison d'au moins 70% en poids par le composé de formule I ; et
b) séchage de la dispersion aqueuse.

10. Composition selon la revendication 9, la dispersion aqueuse du dérivé d'astaxanthine pouvant être obtenue par un procédé comprenant les étapes suivantes :
a1) dissolution du dérivé d'astaxanthine dans un solvant organique qui est constitué de carbone, d'hydrogène et d'oxygène,
a2) mélange de la solution obtenue dans l'étape a1) avec une composition aqueuse qui contient au moins un colloïde de protection protéinique sous forme dissoute.

11. Procédé pour la préparation d'une composition selon l'une quelconque des revendications précédentes, comprenant les étapes suivantes :
a) préparation d'une dispersion aqueuse du dérivé d'astaxanthine, qui contient au moins un colloïde de protection protéinique, le dérivé d'astaxanthine étant constitué à raison d'au moins 70% en poids par le composé de formule I ; et
b) séchage de la dispersion aqueuse.

12. Procédé selon la revendication 11, la préparation de la dispersion aqueuse du dérivé d'astaxanthine comprenant les étapes suivantes :
a1) dissolution du dérivé d'astaxanthine dans un solvant organique qui est constitué de carbone, d'hydrogène et d'oxygène,
a2) mélange de la solution obtenue dans l'étape a1) avec une composition aqueuse qui contient au moins un colloïde de protection protéinique sous forme dissoute.

13. Procédé selon la revendication 11, la préparation de la dispersion aqueuse dans l'étape i) comprenant le mélange du dérivé d'astaxanthine solide avec une solution aqueuse du colloïde de protection et le broyage du mélange.

14. Procédé selon l'une quelconque des revendications 11 à 13, le séchage de la dispersion aqueuse ayant lieu par séchage par pulvérisation ou formulation par pulvérisation.

15. Utilisation d'une composition selon l'une quelconque des revendications 1 à 10 pour la préparation de compositions fourragères, en particulier des compositions fourragères solides, en particulier des compositions fourragères sous forme de pellets.

16. Utilisation d'une composition selon l'une quelconque des revendications 1 à 10 pour la préparation de solutions du dérivé d'astaxanthine dans une huile appropriée pour aliments.

17. Procédé pour la préparation de compositions fourragères, en particulier de compositions fourragères solides, en particulier de pellets, comprenant :
α) préparation d'une composition sous forme de poudre selon l'une quelconque des revendications 1 à 10 ;
β) incorporation de la composition sous forme de poudre dans une huile liquide appropriée pour aliments avec obtention d'une solution liquide diluée du dérivé d'astaxanthine dans l'huile liquide ; et
γ) mélange de la solution liquide obtenue dans l'étape β) avec les constituants de la composition fourragère ou l'imprégnation d'une composition fourragère solide par la solution liquide obtenue dans l'étape β).
